(19) Europäisches Patentamt
European Patent Office
Office européen des brevets

(11) Veröffentlichungsnummer: **0 044 276**
**A2**

# (12) EUROPÄISCHE PATENTANMELDUNG

(21) Anmeldenummer: 81810276.6

(22) Anmeldetag: 09.07.81

(51) Int. Cl.³: C 07 D 405/06
A 01 N 43/50, A 01 N 43/64

(30) Priorität: 15.07.80 CH 5412/80

(43) Veröffentlichungstag der Anmeldung:
20.01.82 Patentblatt 82/3

(84) Benannte Vertragsstaaten:
AT BE CH DE FR GB IT LI NL

(71) Anmelder: CIBA-GEIGY AG
Patentabteilung Postfach
CH-4002 Basel(CH)

(72) Erfinder: Kunz, Walter, Dr.
Im Goldbrunnen 55
CH-4104 Oberwil(CH)

## (54) 4-(1H-Azolylmethyl)-1,3-dioxolan-5-on-derivate, Verfahren zu deren Herstellung und deren Verwendung als wachstumsregulierende und/oder mikrobizide Mittel.

(57) Es werden neue 4-(1H-Azolylmethyl)-1,3-dioxolan-5-on-derivate der hierin definierten Formel I

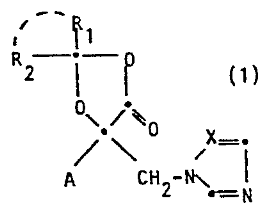

(1)

sowie ihre Herstellung und Verwendung beschrieben. Dabei stehen $R_1$ und $R_2$ für gegebenenfalls substituiertes Alkyl oder Phenyl oder bilden gemeinsam einen drei- bis siebenfliedrigen, gegebenenfalls substituierten carboxyclischen Ring; A ist ein gegebenenfalls substituierter Phenylrest, und X steht für −CH= oder −N=. Die Wirkstoffe der Formel I lassen sich in Form entsprechender Mittel zur Regulierung des Pflanzenwachstums und/oder zur Bekämpfung und/oder Verhütung eines Befalls der Pflanzen durch phytopathogene Mikroorganismen einsetzen.

EP 0 044 276 A2

Croydon Printing Company Ltd.

CIBA-GEIGY AG
Basel (Schweiz)

5-12960/=

# 4-(1H-Azolylmethyl)-1,3-dioxolan-5-on-derivate, Verfahren zu deren Herstellung und deren Verwendung als wachstumsregulierende und/oder mikrobizide Mittel

Die vorliegende Erfindung betrifft substituierte 4-(1H-Azolylmethyl)-1,3-dioxolan-5-on-derivate der Formel I sowie ihre Säureadditionssalze mit organischen und anorganischen Säuren und ihre Metallkomplexsalze. Die Erfindung betrifft ferner die Herstellung dieser Verbindungen sowie wachstumsregulierende und/oder mikrobizide Mittel, die als Wirkstoff eine der Verbindungen der Formel I enthalten. Die Erfindung betrifft auch die Verwendung der Verbindungen der Formel I zur Regulierung des Pflanzenwachstums und/oder zur Bekämpfung von schädlichen Mikroorganismen.

Es werden hierin Verbindungen der Formel I umfasst

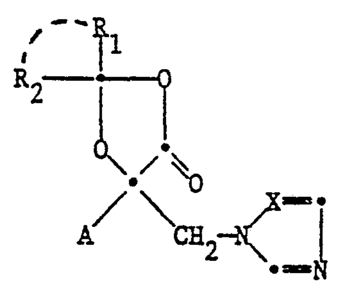

(I)

worin $R_1$ und $R_2$ unabhängig voneinander Wasserstoff, ein gegebenenfalls ein- oder mehrfach durch Halogen, $C_1$-$C_4$-Alkoxy, $C_1$-$C_4$-Alkylthio, Phenyl oder Phenoxy substituiertes $C_1$-$C_8$-Alkyl oder Phenyl bedeuten, wobei die genannten Phenylreste gegebenenfalls ein- oder mehrfach durch Halogen, Nitro, Trifluormethyl, $C_1$-$C_4$-Alkyl oder Methoxy substituiert sind oder $R_1$ und $R_2$ zusammen einen drei- bis siebengliedrigen, carbocyclischen Ring bilden, der gegebenenfalls durch Halogen, $C_1$-$C_4$-Alkyl, $C_1$-$C_4$-Alkoxy oder $C_1$-$C_3$-Alkylthio substituiert ist, A einen gegebenenfalls ein- oder mehrfach durch Halogen, Trifluormethyl, Nitro, $C_1$-$C_4$-Alkyl, $C_1$-$C_4$-Alkoxy oder Phenyl sub-

stituierten Phenylrest darstellt, X -CH= oder -N= ist, unter Einschluss ihrer Säureadditionssalze mit organischen oder anorganischen Säuren, sowie ihren Metallkomplex-Salzen.

Die Wirkstoffe der Formel I umfassen demgemäss die freien Verbindungen, ihre Säureadditionssalze und ihre Metallkomplexe.

Unter Alkyl oder Alkylanteil eines anderen Substituenten sind je nach Zahl der angegebenen C-Atome beispielsweise folgende Gruppen zu verstehen: Methyl, Aethyl, Propyl, Butyl, Pentyl, Hexyl, Heptyl oder Octyl sowie ihre Isomeren, wie z.B. Isopropyl, Isobutyl, tert.-Butyl, Isopentyl usw. Unter einem drei- bis siebengliedrigen, carbocyclischen Ring sind z.B. Cyclopropyl, Cyclobutyl, Cyclopentyl, Cyclohexyl oder Cycloheptyl zusammengefasst. Halogen steht in der Regel für Fluor, Chlor, Brom oder Jod, vorzugsweise für Chlor oder Brom.

Beispiele salzbildender Säuren sind anorganische Säuren: Halogenwasserstoffsäuren wie Fluorwasserstoffsäure, Chlorwasserstoffsäure, Bromwasserstofffäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode des Periodensystems. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten.

Die Verbindungen der Formel I sind biologisch aktive Substanzen, die sich sowohl zur Beeinflussung des Pflanzenwachstums als auch zur Bekämpfung schädlicher, insbesondere phytopathogener Mikroorganismen einsetzen lassen.

Die Wirkstoffe der Formel I eröffnen damit die Möglichkeit, gezielt den Pflanzenwuchs zu regulieren und gleichzeitig die behandelten Pflanzen vor Krankheitserregern zu schützen. Die 1,2,4-Triazolylderivate im Umfang der Formel I sind bevorzugt.

Aufgrund ihrer ausgeprägten wuchsregulierenden und/oder mikrobiziden Wirkung sind diejenigen Substanzen der Formel I bevorzugt, die folgende Substituententypen oder Kombinationen dieser untereinander aufweisen.
Bei $R_1$ und $R_2$ unabhängig voneinander:

a) Wasserstoff, $C_1$-$C_8$-Alkyl, gegebenenfalls durch Halogen, Nitro, Trifluormethyl, Methyl oder Ethyl substituiertes Phenyl, Benzyl;
oder besonders:
b) Wasserstoff, $C_1$-$C_5$-Alkyl, Phenyl;
insbesondere:
c) $C_1$-$C_5$-Alkyl.

Bei A:

a) gegebenenfalls ein- oder mehrfach durch Halogen, Trifluormethyl, Nitro, $C_1$-$C_4$-Alkyl, $C_1$-$C_4$-Alkoxy oder Phenyl substituiertes Phenyl;

oder besonders:

b) gegebenenfalls ein- oder mehrfach durch Halogen, $C_1$-$C_3$-Alkyl, Methoxy oder Phenyl substituiertes Phenyl;

insbesondere:

c) Phenyl, 2,4-Dichlorphenyl, 2,4-Dibromphenyl, 2-Chlor-4-fluorphenyl, 4-Chlorphenyl, 4-Bromphenyl.

Bei X: -CH= oder insbesondere -N=.

Eine bevorzugte Gruppe von wuchsregulierenden und/oder mikrobiziden Wirkstoffen besteht aus Verbindungen der Formel I, worin $R_1$ und $R_2$ unabhängig voneinander für Wasserstoff, $C_1$-$C_8$-Alkyl, ein gegebenenfalls durch Halogen, Nitro, Trifluormethyl, Methyl oder Ethyl substituiertes Phenyl oder Benzyl stehen, A ein gegebenenfalls ein- oder mehrfach durch Halogen, Trifluormethyl, Nitro, $C_1$-$C_4$-Alkyl, $C_1$-$C_4$-Alkoxy oder Phenyl substituiertes Phenyl bedeutet und X für -CH= oder -N= steht.

Eine weitere bevorzugte Gruppe von wuchsregulierenden und/oder mikrobiziden Wirkstoffen besteht aus Verbindungen der Formel I, worin $R_1$ und $R_2$ unabhängig voneinander für Wasserstoff, $C_1$-$C_5$-Alkyl oder Phenyl stehen, A ein gegebenenfalls ein- oder mehrfach durch Halogen, $C_1$-$C_3$-Alkyl, Methoxy oder Phenyl substituiertes Phenyl bedeutet und X für -CH= oder -N= steht.

Eine besonders bevorzugte Gruppe von wuchsregulierenden und/oder mikrobiziden Wirkstoffen besteht aus Verbindungen der Formel I, worin $R_1$ und $R_2$ unabhängig voneinander für $C_1$-$C_5$-Alkyl stehen, A Phenyl, 2,4-Dichlorphenyl, 2,4-Dibromphenyl, 2-Chlor-4-fluorphenyl, 4-Chlorphenyl oder 4-Bromphenyl bedeutet und X für -CH= oder -N= steht.

Bevorzugt werden ferner Verbindungen der Formel I, worin $R_1$ und $R_2$ zusammen einen drei- bis siebengliedrigen, gegebenenfalls durch Methoxy substituierten, carbocyclischen Ring bilden.

Folgende Einzelverbindungen sind aufgrund ihrer ausgeprägten wachstumsregulierenden und/oder mikrobziden Wirkung besonders bevorzugt:

4-(1H-1,2,4-Triazolylmethyl)-4-(4-chlorphenyl)-2,2-dimethyl-1,3-dioxolan-5-on

4-(1H-1,2,4-Triazolylmethyl)-4-(4-bromphenyl)-2,2-dimethyl-1,3-dioxolan-5-on

4-(1H-1,2,4-Triazolylmethyl)-4-(phenyl)-2,2-dimethyl-1,3-dioxolan-5-on

4-(1H-1,2,4-Triazolylmethyl)-4-(2,4-dichlorphenyl)-2,2-dimethyl-1,3-dioxolan-5-on

4-(1H-Imidazolylmethyl)-4-(4-chlorphenyl)-2,2-dimethyl-1,3-dioxolan-5-on

4-(1H-1,2,4-Triazolylmethyl)-4-(4-chlorphenyl)-2,2-diethyl-1,3-dioxolan-5-on

4-(1H-1,2,4-Triazolylmethyl)-5-(2-chlor-4-fluorphenyl)-2,2-dimethyl-1,3-dioxolan-5-on.

Die Verbindungen der Formel I können nach einer Methode, wie z.B. nachfolgend aufgeführt, hergestellt werden.

In den Formeln II bis VI haben $R_1$ und $R_2$ sowie A und X die unter Formel I angegebenen Bedeutungen. M steht für Wasserstoff oder ein Metallion, vorzugsweise ein Alkali- oder Erdalkalikation und G für eine der üblichen Abgangsgruppen wie z.B. Alkoxy, Benzolsulfonyloxy, p-Brombenzolsulfonyloxy, p-Tosyloxy, Trifluoracetyloxy, Niederalkylsulfonyloxy wie Mesyloxy oder insbesondere für Halogen wie Fluor, Chlor, Brom oder Jod, bevorzugt für Chlor oder Brom.

Das Verfahren zur Herstellung von Verbindungen der Formel I kann beispielsweise über die nachfolgend skizzierten Teilreaktionen A, B, C, D oder E durchgeführt werden:

A.

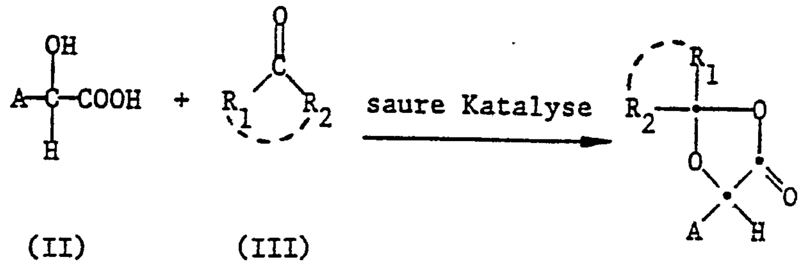


(II) (III) (IV)

B. (IV)

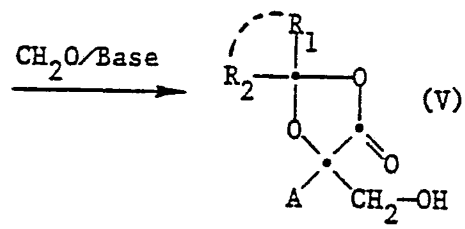


(V)

C. (V)

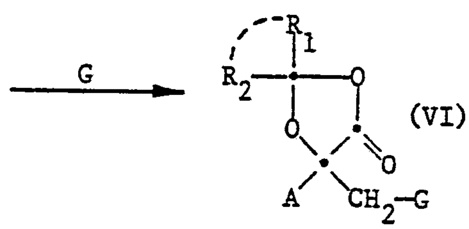


(VI)

D. (VI)

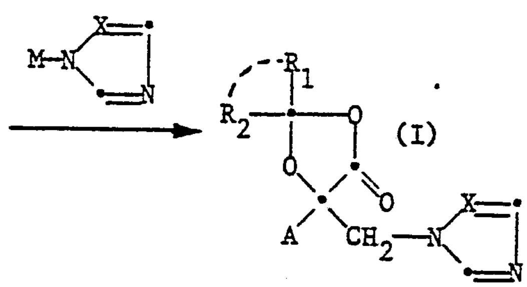

(I)

E. (V)

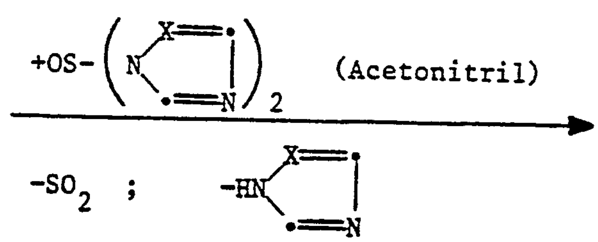


(I)

Bei der <u>Teilreaktion A</u>

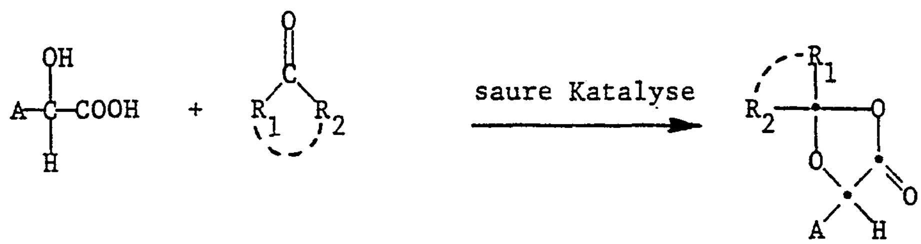


(IV)

(II) (III)

wird eine Säure II [die entweder als Racemat oder in ihrer reinen D- bzw. L-Form vorliegen kann] mit einem gegebenenfalls cyclischen Keton bzw. einem Aldehyd der Formel III zu dem Dioxolanon IV umgesetzt, Hierbei können grundsätzlich Lösungsmittel eingesetzt werden, die sich gegenüber den Reaktionspartnern inert verhalten und zweckmässigerweise mit Wasser Azeotrope bilden. Es eignen sich hierzu beispielsweise aromatische Kohlenwasserstoffe, wie Benzol, Toluol, Xylole oder halogenierte Kohlenwasserstoffe, wie Dichlormethan, Chloroform, Tetrachlorkohlenstoff, 1,2-Dichloräthan, Tetrachloräthylen, Chlorbenzol, aber auch ätherartige Verbindungen, wie tert.-Butylmethyläther, Dioxan und andere. In manchen Fällen kann die Verbindung der Formel III selbst als Lösungsmittel verwendet werden. Die Reaktionstemperatur hängt im wesentlichen von der Wahl des Ketons bzw. Aldehyds III ab und kann beispielsweise zwischen -30° bis 180°C, vorzugsweise -10° bis 140°C liegen. In manchen Fällen ist saure Katalyse empfehlenswert. Als Katalysatoren bzw. Kondensationsmittel eignen sich z.B. Mineralsäuren, wie Halogenwasserstoffsäuren, Schwefelsäure, Phosphorsäure; Sulfosäuren, wie p-Toluolsulfonsäure, saure Ionenaustauscher-Harze usw. Das entstehende Wasser kann nicht nur azeotrop aus dem Reaktionsgemisch entfern werden, sondern auch mit Hilfe von Ionenaustauscher-Harzen, Molekularsieben oder durch Reaktion mit Dicyclohexylcarbodiimid. In manchen Fällen ist es von Vorteil, wenn die Reaktion bei erhöhtem Druck durchgeführt wird.

Im Teilschritt B

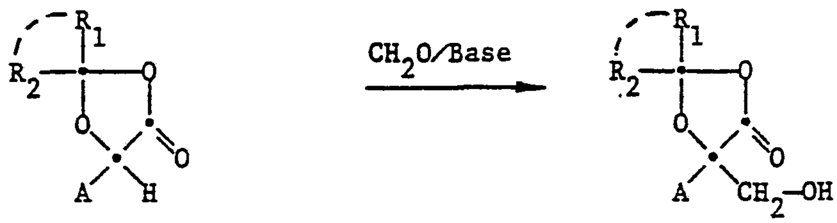


(IV) (V)

reagiert das Dioxolanon IV, vorteilhafterweise in Gegenwart einer Base, mit Formaldehyd zu dem Zwischenprodukt der Formel V ,wobei als Basen tertiäre Amine, wie Trialkylamine, Pyridin, Dimethylanilin usw., oder quarternäre Ammoniumhydroxide, wie Tetramethylammoniumhydroxid und andere, in Frage kommen. Die Base kann hierbei z.B. in äquimolarer Menge, bezogen auf das Produkt IV, eingesetzt werden. Die Teilreaktion B kann z.B. bei Temperaturen zwischen -30° und +80°C durchgeführt werden. Es können dabei Lösungsmittel verwendet werden, die sich gegenüber den Reaktionsteilnehmern inert verhalten, so z.B. ätherartige Verbindungen, wie Tetrahydrofuran, Dioxan usw., oder Nitrile, wie Acetonitril, Propionitril und andere.

Bei der Teilreaktion C

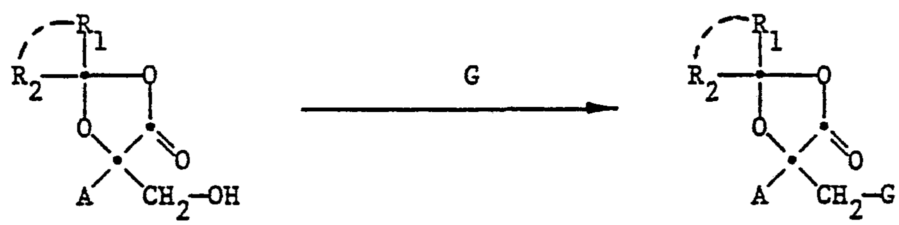


(V) (VI)

wird eine Abgangsgruppe G in das Molekül V eingeführt (G kann z.B. für eine der üblichen Abgangsgruppen wie z.B. Acyloxy, Arylsulfonyloxy wie Benzolsulfonyloxy, p-Brombenzolsulfonyloxy, p-Tosyloxy, oder Alkylsulfonyloxy, vor allem Niederalkylsulfonyloxy wie Mesyloxy, oder für Halogen wie Fluor, Chlor, Brom, Jod, bevorzugt für

Chlor oder Brom, stehen), indem man das Zwischenprodukt V beispielsweise mit Methansulfochlorid, vorteilhafterweise in Gegenwart von tertiären Aminen, wie z.B. Pyridin, Trialkylamin, Collidin usw., zum Zwischenprodukt VI umsetzt. Das Amin wird in mindestens molaren Mengen bezogen auf Verbindung der Formel V eingesetzt. Es können hierbei die gängigen organischen, halogenierten und nicht-halogenierten, aliphatischen und aromatischen Lösungsmittel verwendet werden. Die Abgangsgruppe G kann hierbei wahlweise das Proton oder die OH-Gruppe insgesamt ersetzen.

Zwischenprodukte der Formel VI zeigen zum Teil fungizide Wirkung, so z.B. die Verbindungen der Formel VI mit A = 2,4-Dichlorphenyl und mit $R_1$, $R_2$ = Alkyl, z.B. Methyl.

Im Teilschritt D

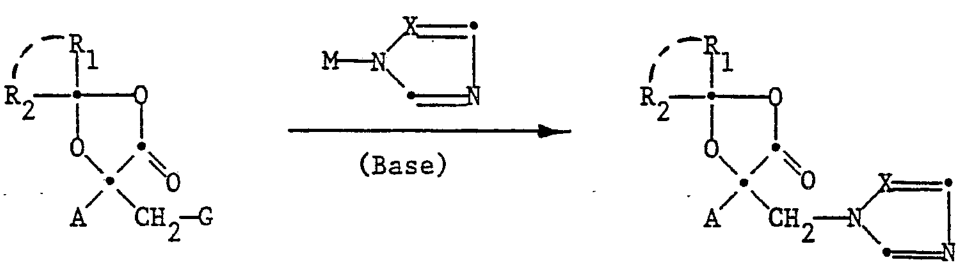


(VI) (I)

wird die Abgangsgruppe G (z.B. $CH_3SO_3$-) durch den Substituenten 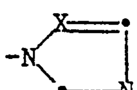 ersetzt, indem z.B. das Produkt VI mit einer Verbindung 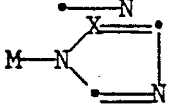 in das Endprodukt der Formel I überführt wird. Die Reaktion kann in den gängigen, inerten, organischen Lösungsmitteln durchgeführt werden, wobei in manchen Fällen der Zusatz von Basen, wie Alkali- oder Erdalkalicarbonaten, Alkalialkoholaten oder Natriumhydrid, angezeigt ist.

Bei der Teilreaktion E

E. (V) 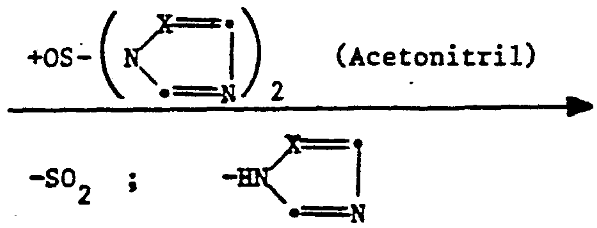

(I)

reagiert ein Alkohol der Formel V wahlweise mit N,N'-Thiocarbonyldi-1,2,4,-triazol oder N,N'-Thiocarbonyldiimidazol unter Eliminierung von $SO_2$ und dem entsprechenden Heterocyclus zu einem Endprodukt der Formel I. Es kann sich dabei als zweckmässig erweisen, die Reaktion in polaren organischen Lösungsmitteln durchzuführen. Beispiele solcher Lösungsmittel sind Aether und ätherartige Verbindungen wie Diäthyläther, Diisopropyläther, t-Butylmethyläther, Dimethoxyäthan, Dioxan, Tetrahydrofuran, Anisol; Nitrile wie Acetonitril, Propionitril; Ester wie Aethylacetat, Butylacetat oder Dialkylamide wie Dimethylamid. Auch Gemische solcher Lösungsmittel untereinander können verwendet werden. Die Umsetzung kann bei Temperaturen ab 20°C aufwärts durchgeführt werden. Zur Beschleunigung der Reaktion ist es jedoch vorteilhaft bei höheren Temperaturen zu arbeiten, beispielsweise zwischen 45° und 90°C oder beim Siedepunkt des Lösungsmittels bzw. Lösungsmittelgemisches.

Die Herstellungsverfahren sind Bestandteil der Erfindung.

Die Ausgangsverbindungen der Formeln II und III sind bekannt und werden nach allgemein bekannten Verfahren hergestellt.

Dioxolanone der Formel IV werden erwähnt in:
Org. Synth. Coll. Vol. 3, 536 (1955)
Bull. Soc. Chim. France 1970, 332
Ber. 68, 303, 609 (1935)
Ber. 72, 319, 798 (1939)

Dioxolanone der Formeln V und VI sind neu. Sie sind speziell für die Herstellung der erfindungsgemässen Verbindungen der Formel I entwickelt worden und stellen darüberhinaus wertvolle Zwischenprodukte zur Synthese von Agarchemikalien dar. Sie sind zum Teil ebenfalls biologisch aktiv und weisen insbesondere pflanzenfungizide Wirkung auf. Die Verbindungen der Formeln V und VI sind daher ebenfalls ein Bestandteil der Erfindung.

Die Herstellung von α-Hydroxycarbonsäuren der Formel II wird beschrieben in:
J. Org. Chim. 33, 2565 (1968)
Org. Synth. Coll. Vol. 3, 326 (1955)
Org. Synth. Coll. Vol. 3, 538 (1955)
2-Methyl-2-äthyl-4-phenyl-1,3-dioxolan-5-on ist aus J. Econ. Entomol. 42, 439 (1949) und 2,2-Dimethyl-4-phenyl-1,3-dioxolan-5-on aus US-PSappl. 70 388 [CA 46, 3209d (1952)] bekannt. Von beiden Verbindungen wird ausschliesslich insektizide Wirkung berichtet.

Die Verbindungen der Formel I besitzen in Nachbarstellung zum Substituenten A ein asymmetrisches Kohlenstoffatom *C und ein weiteres Asymmetriezentrum (*), wenn $R_1$ und $R_2$ verschieden sind. Letzteres ist benachbart den beiden Sauerstoffatomen.

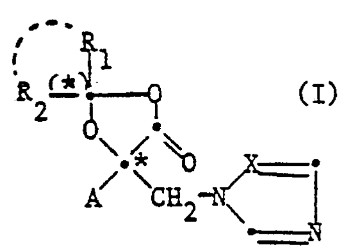

(I)

a) Sind die Reste $R_1$ und $R_2$ identisch, so können die beiden Stereoisomeren nach den üblichen Methoden, z.B. durch fraktionierte Kristallisation der Salze von Verbindungen der Formel I mit einer optisch aktiven Säure, wie z.B. Camphersulfonsäure, in die optischen Antipoden getrennt werden. Zum selben Ziel kann man aber auch mittels einer enantioselektiven Synthese, ausgehend von einer optisch reinen Hydroxysäure II [Teilschritt A], gelangen.

b) Sind die beiden Reste $R_1$ und $R_2$ verschieden, so liegen 4 Stereoisomeren vor. Eine Auftrennung der Diastereomeren-Paare (cis-trans-Isomere) in die Racemate kann, falls dies wünschenswert erscheint, ebenfalls nach den üblichen Methoden, z.B. durch Säulenchromatographie, erfolgen. Für eine weitere Auftrennung der reinen Diastereomeren (cis-trans-Isomeren) in die optisch reinen Enantiomeren kann nach dem unter a) geschilderten Trennverfahren vorgegangen werden.

Die Charakterisierung der einzelnen Konfigurationen kann spektroskopisch z.B. mit Hilfe der NMR-Spektroskopie oder der Röngtenstrukturanalyse erfolgen. Die vorliegende Erfindung bezieht sich auf sämtliche isomeren Verbindungen, ihre Salze und Metallkomplexe.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Die hierin offenbarten Verbindungen der Formel I können zur Regulierung des Wachstums von Pflanzen eingesetzt werden. Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwednung, d.h. vom Entwicklungsstadium des Samens oder der Pflanze, der Art der Applikation sowie insbesondere von den angewendeten Konzentrationen ab. Derartige Effekte sind aber wiederum je nach Pflanzenart verschieden. Die Applikation von Verbindungen der Formel I eröffnet somit die Möglichkeit das Wachstum von Pflanzen in gewünschter Weise zu beeinflussen.

Mit den neuen Wirkstoffen der Formel I bzw. mit den neuen Mitteln wird das vegetative Pflanzenwachstum gedämpft. Durch diese Beeinflussung des Wachstums können die erfindungsgemäss verwendeten Wirkstoffe die Ernteausbeute von Pflanzen wesentlich erhöhen. So erfahren etwa Sojapflanzen und andere Leguminosen wie Bohnen, Erbsen oder Linsen eine Reduktion des vegetativen, zugunsten des generativen Wachstums, wodurch eine unmittelbare Ertragssteigerung erzielt wird. Auch bei weiteren Pflanzenarten, z.B. bei Reben, Getreidesorten, Gräsern und Zierpflanzen wird das vegetative Wachstum in gewünschter Weise gehemmt. Daneben beobachtet man bei den behandelten Pflanzen eine deutliche Stärkung des Stützgewebes.

Eine im Vordergrund stehende Art der Pflanzenbeeinflussung beruht auf der besonderen Eigenschaft der Verbindungen der Formel I, bei bestimmten Pflanzen, insbesondere bei Getreidekulturen, in Aufwandmengen von 0,2 bis 5 kg AS/ha eine gezielte Wuchshemmung hervorzurufen, die bei unveränderter Ertragsleistung die Knickfestigkeit der Pflanzen wesentlich erhöht. Daraus ergibt sich eine wirtschaftlich sehr interessante Methode des Schutzes von Pflanzenkulturen vor Lagerung durch Sturm oder Unwetter. Darüberhinaus gestattet eine gezielte Hemmung des vegetativen Wachstums bei vielen Kulturpflanzen eine dichtere Anpflanzung der Kulturen; dies führt bei gleichem Fruchtansatz und gleicher Anbaufläche zu einer deutlichen Ertragssteigerung. Der Einsatz von Wuchshemmern bewirkt auch eine effektivere Nutzung der Nährstoffe, die nunmehr in verstärktem Masse der Blüten- und Fruchtbildung zugute kommen. Auf diese Weise lassen sich höhere Ernteerträge bei gleichzeitig kleinerem Abfallanteil an vegetativen Pflanzenresten [z.B. Stroh, Kartoffelkraut, Rübenblätter] erzielen.

Hervorzuheben ist auch die Möglichkeit, mit den erfindungsgemässen Stoffen bzw. Mitteln bei verschiedenen Pflanzenarten, insbesondere bei Tabakpflanzen eine Hemmung des unerwünschten Geiztriebwuchses zu erreichen, wenn der Haupttrieb kurz vor der Blüte zum Zwecke der erstrebten Wuchsvergrösserung der Blätter abgeschnitten worden ist.

Im Gegensatz zu den bisher beschriebenen Effekten bei niedrigen Anwendungskonzentrationen, führt eine hohe Dosierung der erfindungsgemässen Verbindungen, z.B. die Applikation von 5 bis 10 kg AS/ha bei einer Reihe mono- und dicotyler Pflanzen zu einer sehr starken Wuchsreduktion oder sogar zu Zwergwuchs. Daraus ergibt sich eine vom Standpunkt der Kulturbodenerhaltung besonders vorteilhafte Methode zur Zurückdrängung von Schadpflanzen, insofern als unabhängig vom Nutzpflanzenwachstum eine gleichmässige, niedrige Pflanzendecke erhalten bleibt, die einer Bodenerosion durch Wind oder Wasser entgegenwirkt. In dieses Einsatzgebiet fällt insbesondere die Wachstumsreduktion bei Gräsern bei der Instandhaltung von reinen Grasbepflanzungen, wie sie als Grünanlagen, in Wohngegenden, auf Industriegelände oder an Autostrassen, Eisenbahndämmen oder Uferböschungen von Gewässern angelegt werden. In all diesen Fällen ist normalerweise ein periodisches Schneiden des Rasens bzw. Grasbewuchses notwendig. Dies ist nicht nur im Hinblick auf Arbeitskräfte und Maschinen sehr aufwendig, sondern birgt im Verkehrsbereich erhebliche Gefahren für das betroffene Personal und die Verkehrsteilnehmer.

Es besteht daher gerade in Gebieten mit grossen Verkehrsnetzen ein dringendes Bedürfnis, die zur Verfestigung von Seitenstreifen und Böschungen an Verkehrswegen notwendige Grasnarbe einerseits zu erhalten und zu pflegen, andererseits aber mit einfachen Massnahmen während der gesamten Vegetationsperiode auf einer mittleren Wuchshöhe zu halten. Dies wird durch Applikation der Verbindungen der Formel I auf sehr günstige Weise erreicht.

Neben den wachstumshemmenden Eigenschaften, bieten Verbindungen der Formel I in manchen Fällen auch die Möglichkeit in den pflanzlichen Stoffwechsel einzugreifen. Durch den Einsatz von Wachstumsregulatoren lässt sich auf diese Weise eine verbesserte Qualität der Ernteprodukte bewirken. So lassen sich neben Grösse und Gewicht z.B. der Proteingehalt bei Leguminosen wie Bohnen, Linsen, Erbsen, insbesondere bei Soja steigern.

Darüberhinaus üben die Aktivsubstanzen der Formel I eine günstige Wirkung auf die Keimfähigkeit, Blütenbildung und Fruchtentwicklung aus. Die Applikation der erfindungsgemässen Wirkstoffe verändert nicht den von genetischen Eigenschaften determinierten Lebenszyklus der Pflanzen im Sinne einer Mutation.

Weiter hat es sich überraschenderweise gezeigt, dass die Aktivsubstanzen der Formel I bzw. entsprechende Mittel, ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Pflanzen ohne diese durch unerwünschte Nebenwirkungen zu beeinflussen. Pflanzen seien im Rahmen vorliegender Erfindung Kulturpflanzen wie Getreide:(Weizen,Gerste, Roggen, Hafer, Reis); Rüben: (Zucker- und Futterrüben); Kern-, Stein und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- oder Naturkautschukgewächse. Pflanzen seien im Rahmen vorliegender Erfindung aber auch alle Arten von sonstigen Grünbewachsungen, seien es Zierpflanzen, Grasflächen, Böschungen oder allgemeine niedrige Bodenbedeckungen, die einer Erosion oder Austrocknung des Bodens entgegenwirken, Bodenbedeckungen wie sie in Baum- und Staudenkulturen (Obstplantagen, Hopfenkulturen, Maisfeldern, Weingärten usw.) erwünscht sind.

Mit den Wirkstoffen der Formel I können an Pflanzen oder an Pflanzenteilen (Früchte, Blüten, Laubwerk, Stengel, Knollen, Wurzeln), der genannten und verwandter Nutzkulturen auch auftretende Mikroorganismen eingedämmt oder vernichtet werden, wobei auch später hinzuwachsende Pflanzenteile von derartigen Mikroorganismen verschont bleiben.

Die Wirkstoffe der Formel I sind unter anderen gegen die den folgenden Klassen angehörenden phytopathogenen Pilze wirksam: Gegen die zur Klasse der Phycomycetes gehörenden Oomycetes (z.B. Phytophthora); gegen Fungi imperfecti wie z.B. Cercospora, insbesondere aber gegen die zur Klasse der Ascomycetes gehörenden Erysiphe- und Venturia-Erreger sowie gegen Basidiomycetes, vor allem Rostpilze wie Puccinia.

Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Der weitaus grösste Vorteil für den praktischen Einsatz von Wirkstoffen der Formel I auf dem Agrarsektor besteht nun darin, dass zwei in der Praxis bedeutungsvolle Probleme, nämlich einerseits Kräftigung der Pflanze im Sinne einer morphologischen Stabilisierung

und andererseits ihr Schutz gegen Krankheiten, im Dosierungsbereich von 0,05 bis 5 kg AS/ha, bevorzugt 0,2 bis 2,5 kg AS/ha auf einfache Weise durch Applikation einer einzigen Aktivsubstanz bzw. eines Mittels, gleichzeitig gelöst werden. Der Einsatz der erfindungsgemässen Wirkstoffe führt somit zu einer wesentlichen Verringerung der Umweltbelastung. Hinzu kommt als unmittelbare Auswirkung die in den meisten Fällen zu beobachtende deutliche Ertragssteigerung bei Kulturpflanzen.

Die Erfindung betrifft somit die Verwendung der Wirkstoffe der Formel I zur Regulierung des Pflanzenwachstums und/oder zur Bekämpfung und/oder Verhütung eines Befalls von Mikroorganismen.

Wirkstoffe der Formel I können gleichzeitig oder nacheinander mit weiteren Wirkstoffen auf die zu behandelnde Fläche oder Pflanze gegeben werden. Diese Wirkstoffe können sowohl Dünggemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Unter Tensiden sollen hierbei grenzflächen- oder oberflächenaktive Verbindungen verstanden werden, die meistens in einer Flüssigkeit gelöst oder dispergiert sind und bevorzugt an Grenzflächen adsorbiert werden. Ein Tensidmolekül besitzt mindestens eine Gruppe,

die eine Affinität zu Substanzen starker Polarität aufweist - wodurch im allgemeinen die Löslichkeit in Wasser verursacht wird - und mindestens eine weitere Gruppe mit geringer Affinität zu Wasser. Tenside sind somit Moleküle mit einem lipophilen = hydrophoben, d.h. wasserabweisenden oder fettfreundlichen Molekülteil, meist einem Kohlenwasserstoffrest mit Alkyl- und/oder Arylkomponenten und ausserdem mit einem hydrophilen = lipophoben, d.h. wasserfreundlichen und fettabweisenden Molekülteil, z.B. einem Perfluoralkylrest. Die in der Praxis gebrauchten Produkte sind meistens Mischungen solcher Verbindungen. Tenside ermöglichen nicht nur eine feine Verteilung der Aktivsubstanz in einem flüssigen z.B. wässrigen Medium, sondern auch eine erhöhte Benetzbarkeit der Pflanzen, dies führt zu einer Reduktion des Wirkstoffanteils im gebrauchsfähigen Mittel und damit zu einer geringeren Umweltbelastung.

Der Gehalt an Wirkstoff in handelsfähigen Mitteln liegt zwischen 0,01 bis 90 Gewichtsprozent, der Gehalt an Zuschlagstoffen 10 bis 99,99 Gewichtsprozent,wobei sich im allgemeinen unter den Zuschlagstoffen 0 bis 30 Gewichtsprozent eines Tensides befinden.

Die Erfindung umfasst auch Mittel, die als mindestens eine Aktivsubstanz eine Verbindung der Formel I enthalten, sowie die Verwendung der Mittel zur Regulierung des Pflanzenwachstums und/oder zur Bekämpfung und/oder Verhütung eines Befalls von Mikroorganismen. Darüberhinaus schliesst die vorliegende Erfindung auch die Herstellung der Mittel ein, die gekennzeichnet ist durch das innige Vermischen der Aktivsubstanz mit einem oder mit mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

In den nachfolgenden Beispielen sind Temperaturen in Celsiusgraden angegeben, Prozentangaben beziehen sich stets auf Gewicht.

Sofern nicht besonders vermerkt, ist bei der Nennung eines Wirkstoffes der Formel I stets das racemische bzw. Diastereomerengemisch gemeint. Erfolgt keine gezielte Synthese reiner Isomeren, so fällt ein Produkt der Formel I als Gemisch aller möglichen Isomeren an.

Herstellungsbeispiele:

Beispiel 1:

a) Herstellung eines Ausgangsstoffes

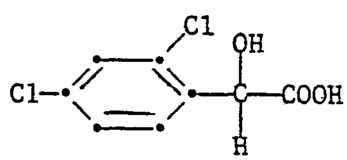

(VII)

2,4-Dichlormandelsäure.

Man lässt innerhalb von 30 Minuten 51,6 g (0,2 Mol) 2,4,1',1'-Tetrachloracetophenon bei 60° zu einer Lösung von 29,4 g Natriumhydroxid in 300 ml Wasser tropfen, wobei die Reaktionstemperatur durch entsprechende Tropfgeschwindigkeit auf 60° gehalten wird. Anschliessend wird noch eine Stunde weitergerührt, filtriert und 37%ige Chlorwasserstoffsäure zugegeben. Man extrahiert mehrmals mit Diäthyläther, wäscht die vereinigten Extrakte mit Wasser, trocknet sie über Natriumsulfat. Dann wird filtriert und bis zur Trockne eingedampft. Man erhält farblose Kristalle vom Smp. 107-111°.

b) Herstellung des Zwischenprodukts

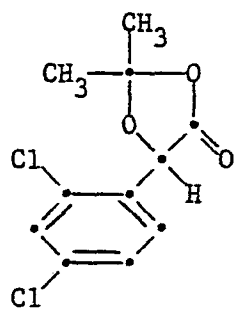

(VIII)

2,2-Dimethyl-4(2,4-dichlorphenyl)-1,3-dioxolan-5-on.

Man kühlt eine Lösung aus 221 g (1 Mol) 2,4-Dichlormandelsäure und 450 ml Aceton auf -10 bis -20° ab und lässt 100 g (54,3 ml) 98%ige Schwefelsäure zutropfen. Anschliessend rührt man noch zwei Stunden bei -20°. Man giesst dann das Reaktionsgemisch auf eine Lösung aus 200 g Natriumcarbonat in 1,8 Liter Eiswasser, filtriert den anfallenden Niederschlag ab, wäscht ihn mit Wasser, löst ihn in Methylenchlorid und trocknet die Lösung mit Natriumsulfat. Nach Entfernen des Lösungsmittels schmilzt das Produkt bei 57-58°.

c) Herstellung des Zwischenprodukts

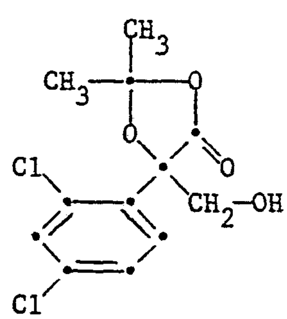

(IX)

2,2-Dimethyl-4-(2,4-dichlorphenyl)-4-hydroxymethyl-1,3-dioxolan-5-on.

Man löst 104,4 g (0,4 Mol) 2,2-Dimethyl-4(2,4-dichlorphenyl)-1,3-dioxolan-5-on in 700 ml Pyridin und gibt 48 g Paraformaldehyd hinzu, anschliessend lässt man 40 ml einer 40%igen methanolischen Lösung

von Tetramethylammoniumhydroxid langsam zutropfen, rührt noch 12 Stunden weiter, kühlt auf -5° ab und lässt bei 0° bis -5° solange eine Lösung aus 100 ml Eisessig und 400 ml Pyridin zutropfen, bis sich ein pH-Wert von 6,5 bis 7 einstellt. Man giesst nun die Lösung auf Eiswasser und extrahiert mit Methylenchlorid, trocknet den Extrakt über Natriumsulfat und bringt das Produkt durch Zugabe von Diäthyläther zur Kristallisation. Der kristalline Niederschlag wird mit wenig kaltem Diäthyl- und Petroläther gewaschen; er schmilzt bei 182-185°.

d) Herstellung des Zwischenprodukts

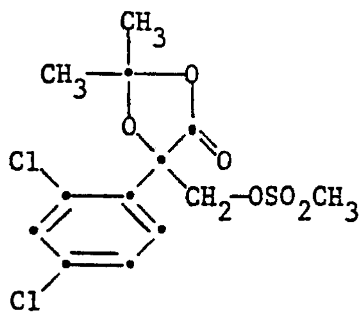

(X)

2,2-Dimethyl-4-(2,4-dichlorphenyl)-4-methansulfonyloxymethyl-1,3-dioxolan-5-on.

Man löst 63,5 g (0,22 Mol) 2,2-Dimethyl-4(2,4-dichlorphenyl)-4-hydroxymethyl-1,3-dioxolan-5-on in 400 ml Pyridin und versetzt die Lösung bei 0 bis 5° mit 28,9 g Methansulfonsäurechlorid. Man rührt die entstehende Suspension noch zwei Stunden bei 0° bis 5° weiter, giesst sie anschliessend auf Eiswasser und extrahiert mit Methylenchlorid. Man wäscht den Extrakt mit Wasser, trocknet ihn über Natriumsulfat und dampft ihn bis zur Trockne ein. Man bringt das verbleibende Harz durch Zugabe von wenig Diäthyläther/Hexan zur Kristallisation und filtriert das Produkt ab; es schmilzt bei 98-100°.

e) Herstellung des Endprodukts

(XI)

(Verb.Nr. 1.3)

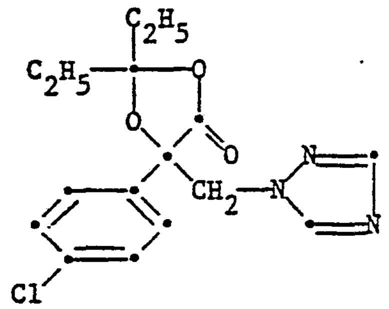

4-(1H-1,2,4-Triazolylmethyl)-4-(4-chlorphenyl)-2,2-diäthyl-1,3-dioxolan-5-on.

Unter einer Stickstoffatmosphäre legt man unter Rühren 5,2 g Natriumhydrid (55%ige Oeldispersion) in 50 ml absolutem Dimethylformamid vor und versetzt diese tropfenweise mit 16,6 g 1,2,4-Triazol, wobei elementarer Wasserstoff entweicht. Man erwärmt das Reaktionsgemisch zwei Stunden lang auf 70°, kühlt es auf Raumtemperatur ab, lässt eine Lösung aus 29 g (0,08 Mol) 2,2-Diäthyl-4-(chlorphenyl)-4-methansulfonyloxymethyl-1,3-dioxolan-5-on in 50 ml Dimethylformamid zutropfen. Man erwärmt das Gemisch anschliessend bis zur vollständigen Umsetzung. Man kühlt die Lösung ab, versetzt sie mit 100 ml Eiswasser und giesst das Gemisch auf Eiswasser. Man filtriert den entstandenen Niederschlag ab, löst ihn in Methylenchlorid, wäscht die Lösung mit Wasser und trocknet sie über Natriumsulfat. Beim Eindampfen bildet sich ein Harz, das durch Zugabe von wenig Diäthyläther auskristallisiert. Man filtriert es ab und wäscht es mit Petroläther. Die Verbindung IX schmilzt bei 105-108°.

Auf analoge Weise werden folgende Endprodukte der Formel I sowie Zwischenprodukte der jeweiligen Formel hergestellt:

Tabelle 1 (Endprodukte)

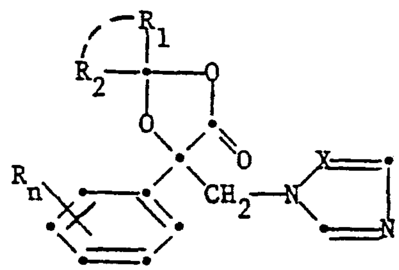

(XII)

| Verb.Nr. | $R_1$ | $R_2$ | $R_n$ | X | Physikal.Konst. |
|---|---|---|---|---|---|
| 1.1 | $CH_3$ | $CH_3$ | H | N | Smp. 115-116° |
| 1.2 | $CH_3$ | $CH_3$ | 2,4-Di-Cl | N | Smp. 153-155° |
| 1.3 | $C_2H_5$ | $C_2H_5$ | 4-Cl | N | Smp. 105-108° |
| 1.4 | $C_2H_5$ | $C_2H_5$ | 4-Cl | CH | Smp. 84-88° |
| 1.5 | $CH_3$ | $CH_3$ | 4-Br | N | Smp. 125-128° |
| 1.6 | $CH_3$ | $C_4H_9$-n | 2,4-Di-Cl | N | Smp. 78-85° |
| 1.7 | $CH_3$ | $C_2H_5$ | 2,4-Di-Cl | N | Smp. 138-142° |

| Verb.Nr. | $R_1$ | $R_2$ | $R_n$ | X | Physik.Konst. |
|---|---|---|---|---|---|
| 1.8 | $CH_3$ | $C_3H_7$-i | 2,4-Di-Cl | N | Smp. 68-71° |
| 1.9 | $C_2H_5$ | $C_2H_5$ | 2,4-Di-Cl | N | Smp.126 - 128° |
| 1.10 | $CH_3$ | $C_5H_{11}$-n | 2,4-Di-Cl | N | |
| 1.11 | $C_5H_{11}$-n | $C_5H_{11}$-n | 2,4-Di-Cl | N | |
| 1.12 | $CH_3(CH_2)_8$- | $CH_3(CH_2)_8$- | 2,4-Di-Cl | N | |
| 1.13 | $CH_3$ | $CH_3$ | 2-Cl, 4-F | N | Smp. 144-150° |
| 1.14 | $CH_3(CH_2)_8$- | $CH_3(CH_2)_8$- | 2,4-Di-Cl | CH | |
| 1.15 | H | H | 2,4-Di-Cl | CH | |
| 1.16 | $C_3H_7$(n) | $C_3H_7$(n) | 2,4-Di-Cl | N | Smp.135 -139° |
| 1.17 | H | H | H | N | Harz |
| 1.18 | H | 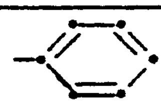 | 2,4-Di-Cl | N | |
| 1.19 | H | 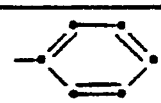 | 2,4-Di-Cl | CH | |

| Verb. Nr. | $R_1$ | $R_2$ | $R_n$ | X |
|---|---|---|---|---|
| 1.20 | H | 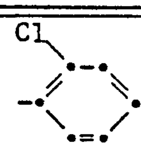 | 2,4-Di-Cl | N |
| 1.21 | H | 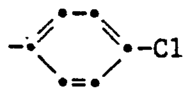 | 2,4-Di-Cl | N |
| 1.22 | H | 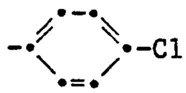 | 2,4-Di-Cl | CH |
| 1.23 | H | 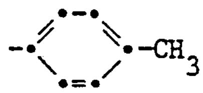 | 2,4-Di-Cl | N |
| 1.24 | H | 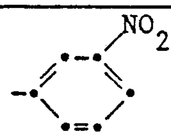 | 2,4-Di-Cl | N |
| 1.25 | H | 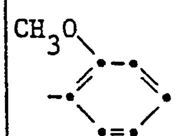 | 2,4-Di-Cl | N |
| 1.26 | H | 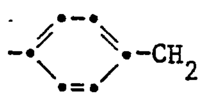 | 2,4-Di-Cl | CH |
| 1.27 | $CH_3$ | 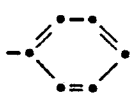 | 2,4-Di-Cl | N |
| 1.28 | $CH_3$ | 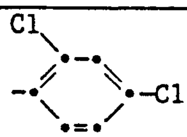 | 2,4-Di-Cl | N |
| 1.29 | $CH_3$ | 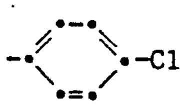 | 2,4-Di-Cl | N |

| Verb. Nr. | $R_1$ | $R_2$ | $R_n$ | X | Physik.Konst. |
|---|---|---|---|---|---|
| 1.30 | $CH_3$ | 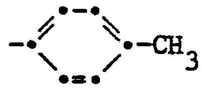 | 2,4-Di-Cl | N | |
| 1.31 | $CH_3$ | 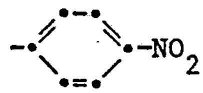 | 2,4-Di-Cl | N | |
| 1.32 | $CH_3$ | 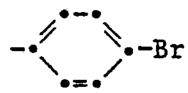 | 2,4-Di-Cl | CH | |
| 1.33 | $CH_3$ | 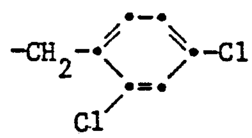 | 2,4-Di-Cl | N | |
| 1.34 | $CH_3$ | 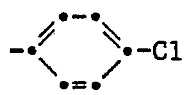 | 2,4-Di-Cl | CH | |
| 1.35 | H | 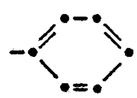 | 4-Cl | N | Smp. 120 - 122° |
| 1.36 | H | 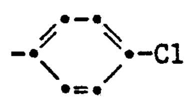 | 4-$CH_3$ | N | |
| 1.37 | H | 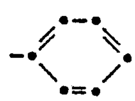 | 2,5-Di-$CH_3$ | N | |
| 1.38 | $CH_3$ | $CH_3$ | 4-Cl | N | |
| 1.39 | H | 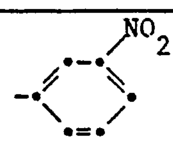 | 2-F | CH | |
| 1.40 | H | 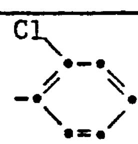 | 4-$OCH_3$ | N | |

| Verb. Nr. | $R_1$ | $R_2$ | $R_n$ | X |
|---|---|---|---|---|
| 1.41 | H | H | 2,5-Di-$CH_3$ | N |
| 1.42 | H | H | 2,5-Di-$CH_3$ | CH |
| 1.43 | $CH_3$ | $C_5H_{11}$-n | 2,5-Di-$CH_3$ | N |
| 1.44 | $CH_3$ | 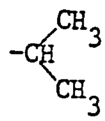 | 4-Cl | N |
| 1.45 | H | 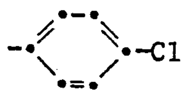 | 4-Cl | N |
| 1.46 | $CH_3$ | 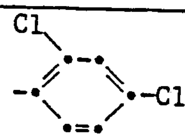 | 4-Cl | N |
| 1.47 | $C_2H_5$ | 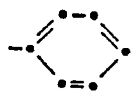 | 4-Cl | N |
| 1.48 | H | $CCl_3$ | 4-Cl | N |
| 1.49 | H | $CBr_3$ | 2,4-Di-Cl | N |
| 1.50 | 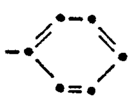 | 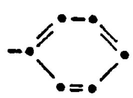 | 2,4-Di-Cl | N |
| 1.51 | $CH_3$ | 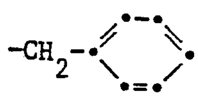 | 2,4-Di-Cl | N |
| 1.52 | $CH_3$ | 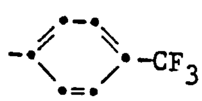 | 2,4-Di-Cl | N |

| Verb. Nr. | $R_1$ | $R_2$ | $R_n$ | X |
|---|---|---|---|---|
| 1.53 | $CH_3$ | $CH_3$ | 4-$NO_2$ | N |
| 1.54 | H | $-CH_2-$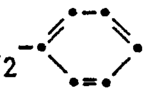 | 2,4-Di-Cl | N |
| 1.55 | H | $C_3H_7$-n | 2,4-Di-Cl | N |
| 1.56 | H | 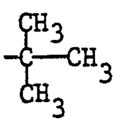 | 2,4-Di-Cl | N |
| 1.57 | $CH_3$ | $-CH_2-O-$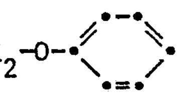 | 2,4-Di-Cl | N |
| 1.58 | $CH_3$ | $-CH_2-O-$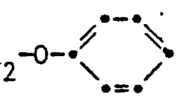 | 2,4-Di-Cl | CH |
| 1.59 | $CH_3$ | $-CH_2-O-CH_3$ | 2,4-Di-Cl | N |
| 1.60 | $CH_3$ | $-CH_2-O-CH_3$ | 4-Cl | CH |
| 1.61 | $CH_3$ | $CH_3$ | 2-$OCH_3$ | N |
| 1.62 | $CH_3$ | $CH_3$ | 4-$OCH_3$ | N |
| 1.63 | $CH_3$ | $CH_3$ | 2-$OCH_3$ | CH |
| 1.64 | $C_2H_5$ | $C_2H_5$ | 2-$OCH_3$ | N |
| 1.65 | $CH_3$ | $CH_3$ | 2,4-Di-$OCH_3$ | N |
| 1.66 | $CH_3$ | $CH_3$ | 2-$OC_2H_5$ | N |

| Verb. Nr. | $R_1$ | $R_2$ | $R_n$ | X |
|---|---|---|---|---|
| 1.67 | H | H | 2-$OCH_3$ | CH |
| 1.68 | H | H | 2-$OC_3H_7$-i | N |
| 1.69 | $CH_3$ | $CH_3$ | 2-$OC_3H_7$-i | N |
| 1.70 | $C_2H_5$ | $C_2H_5$ | 4-$OC_3H_7$-i | N |

Tabelle 1a (Endprodukte)

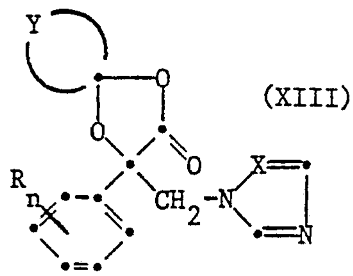

(XIII)

| Verb. Nr. | $R_n$ | Y | X | Physik. Konst. |
|---|---|---|---|---|
| 1.71 | 2,4-Di-Cl | 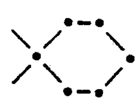 | N | Smp. 132 - 135° |
| 1.72 | 2,4-Di-Cl | 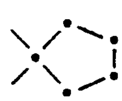 | N | |
| 1.73 | 2,4-Di-Cl | 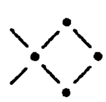 | N | |
| 1.74 | 2,4-Di-Cl | 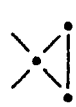 | N | |

| Verb. Nr. | $R_n$ | Y | X | Physikal.Konst. |
|---|---|---|---|---|
| 1.75 | 2,4-Di-Cl | 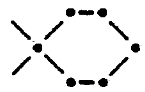 | CH | |
| 1.76 | 4-Cl | 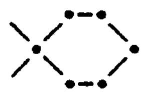 | N | |
| 1.77 | 2,5-Di-Cl | 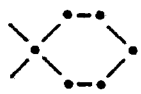 | N | |
| 1.78 | 4-Br | 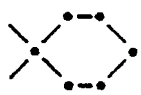 | N | Smp. 137 - 138° |

Tabelle 2 (Zwischenprodukte)

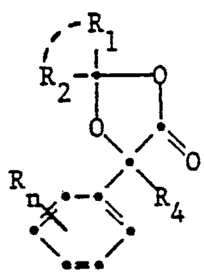

(XIV)

| Verb. Nr. | $R_1$ | $R_2$ | $R_n$ | $R_4$ | Physikal. Konst. |
|---|---|---|---|---|---|
| 2.1 | $CH_3$ | $CH_3$ | H | H | Smp. 42-43° |
| 2.2 | $CH_3$ | $CH_3$ | H | $CH_2OH$ | viskoses Oel |
| 2.3 | $CH_3$ | $CH_3$ | H | $CH_2OSO_2CH_3$ | Smp. 65-71° |
| 2.4 | $CH_3$ | $CH_3$ | 2,4-Di-Cl | H | Smp. 57-58° |
| 2.5 | $CH_3$ | $CH_3$ | 2,4-Di-Cl | $CH_2OH$ | Smp. 181-182° |

| Verb.Nr. | $R_1$ | $R_2$ | $R_n$ | $R_4$ | Physikal. Konst. |
|---|---|---|---|---|---|
| 2.6 | $CH_3$ | $CH_3$ | 2,4-Di-Cl | $CH_2OSO_2CH_3$ | Smp. 95-100° |
| 2.7 | $C_2H_5$ | $C_2H_5$ | 4-Cl | H | Sdp. 130-132°/ 0,7 Torr |
| 2.8 | $C_2H_5$ | $C_2H_5$ | 4-Cl | $CH_2OH$ | Smp. 83 - 84° |
| 2.9 | $C_2H_5$ | $C_2H_5$ | 4-Cl | $CH_2OSO_2CH_3$ | Smp. 60 - 63° |
| 2.10 | $CH_3$ | $C_4H_9$-n | 2,4-Di-Cl | H | viskoses Oel |
| 2.11 | $CH_3$ | $C_4H_9$-n | 2,4-Di-Cl | $CH_2OH$ | Harz |
| 2.12 | $CH_3$ | $C_4H_9$-n | 2,4-Di-Cl | $CH_2OSO_2CH_3$ | Harz |
| 2.13 | $C_3H_7$-n | $C_3H_7$-n | 2,4-Di-Cl | H | Smp.41-43° |
| 2.14 | $C_3H_7$-n | $C_3H_7$-n | 2,4-Di-Cl | $CH_2OH$ | Harz |
| 2.15 | $C_3H_7$-n | $C_3H_7$-n | 2,4-Di-Cl | $CH_2OSO_2CH_3$ | Smp. 49-51° |
| 2.16 | $C_2H_5$ | $C_2H_5$ | 2,4-Di-Cl | H | Sdp.146-148°/ 0,8 Torr |
| 2.17 | $C_2H_5$ | $C_2H_5$ | 2,4-Di-Cl | $CH_2OH$ | Smp. 129-132° |
| 2.18 | $C_2H_5$ | $C_2H_5$ | 2,4-Di-Cl | $CH_2OSO_2CH_3$ | Smp. 75-77° |
| 2.19 | H | $CCl_3$ | 2,4-Di-Cl | H | Smp. 90-94° |
| 2.20 | $CH_3$ | $CH_3$ | 4-Br | H | Smp. 62-64° |
| 2.21 | $CH_3$ | $CH_3$ | 4-Br | $CH_2OH$ | Smp. 123-127° |
| 2.22 | $CH_3$ | $CH_3$ | 4-Br | $CH_2OSO_2CH_3$ | Smp.73-76° |
| 2.23 | $CH_3$ | $CH_3$ | 4-Br | $CH_2O$-Tosyl | Smp. 67-70° |
| 2.24 | $CH_3$ | $C_3H_7$-i | 2,4-Di-Cl | H | Harz |

| Verb.Nr. | $R_1$ | $R_2$ | $R_n$ | $R_4$ | Physikal. Konst. |
|---|---|---|---|---|---|
| 2.25 | $CH_3$ | $C_3H_7$-i | 2,4-Di-Cl | $CH_2OH$ | Harz |
| 2.26 | $CH_3$ | $C_3H_7$-i | 2,4-Di-Cl | $CH_2OSO_2CH_3$ | Harz |
| 2.27 | H | $C_6H_5$ | 4-Cl | H | Smp.78-81° |
| 2.28 | H | $C_6H_5$ | 4-Cl | $CH_2OH$ | Harz |
| 2.29 | H | $C_6H_5$ | 4-Cl | $CH_2OSO_2CH_3$ | Harz |
| 2.30 | $CH_3$ | $CH_3$ | 2,4-Di-$CH_3$ | H | Smp.61-63° |
| 2.31 | H | H | H | H | Oel |
| 2.32 | H | H | H | $CH_2OH$ | Harz |
| 2.33 | H | H | H | $CH_2OSO_2CH_3$ | Harz |
| 2.34 | $-(CH_2)_5-$ | | 2,4-Di-Cl | H | Smp.60-66° |
| 2.35 | $-(CH_2)_5-$ | | 2,4-Di-Cl | $CH_2OH$ | Harz |
| 2.36 | $-(CH_2)_5-$ | | 2,4-Di-Cl | $CH_2OSO_2CH_3$ | Harz |
| 2.37 | $-(CH_2)_5-$ | | 4-Br | H | Smp.79-81° |
| 2.38 | $-(CH_2)_5-$ | | 4-Br | $CH_2OH$ | Harz |
| 2.39 | $-(CH_2)_5-$ | | 4-Br | $CH_2OSO_2CH_3$ | Smp. 65-68° |
| 2.40 | $-(CH_2)_4-$ | | 2,4-Di-Cl | H | Smp.59-61° |
| 2.41 | $-(CH_2)_4-$ | | 2,4-Di-Cl | $CH_2OH$ | Harz |
| 2.42 | $-(CH_2)_4-$ | | 2,4-Di-Cl | $CH_2OSO_2CH_3$ | Harz |

Zur Applikation können die Verbindungen der Formel I in den folgenden Aufarbeitungsformen vorliegen.

Formulierungsbeispiele

Beispiel 2: Feste Aufarbeitungsformen:

Stäube- und Streumittel enthalten im allgemeinen bis zu 100 % des Wirkstoffes. Ein 5 %iges Stäubemittel kann beispielsweise auf 5 Teilen des Wirkstoffs und 95 Teilen eines Zuschlagstoffes wie Talkum bestehen oder aus 5 Teilen Wirkstoff,3 Teilen hochdiesperser Kieselsäure und 92 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

Granulate wie Umhüllungsgranulate,Imprägniergranulate, Homogengranulate und Pellets[=Körner] enthalten üblicherweise 1 bis 80% des Wirkstoffs. So kann sich ein 5 %iges Granulat z.B. aus 5 Teilen des Wirkstoffs, 0,25 Teilen epoxidiertem Pflanzenöl, 0,25 Teilen Cetylpolyglykolether, 3,50 Teilen Polyethylenglykol und 91 Teilen Kaolin (bevorzugte Korngrösse 0,3-0,8 mm) zusammensetzen. Man kann bei der Herstellung des Granulates wie folgt vorgehen:

Die Aktivsubstanz wird mit einem oberflächenaktiven Mittel z.B. epoxidiertem Pflanzenöl vermischt und mit 6 Teilen Aceton gelöst, hierauf wird Polyethylenglykol und Cetylpolyglykolether zugesetzt. Die so erhaltene Lösung wird auf Kaolin aufgesprüht, und anschliessend wird das Aceton im Vakuum verdampft. Ein derartiges Mikrogranulat wird vorteilhaft zur Wuchshemmung bei Getreide und zur präventiven und kurativen Bekämpfung von phytopathogenen Mikroorganismen eingesetzt.

Beispiel 3: Flüssige Aufarbeitungsformen:

Man unterscheidet im allgemeinen zwischen Wirkstoffkonzentraten, die in Wasser dispergierbar oder löslich sind und Aerosolen.

Zu den in Wasser dispergierbaren Wirkstoffkonzentraten zählen z.B. Spritzpulver (wettable powders) und Pasten, die üblicherweise in den Handelspackungen 25-90% und in gebrauchsfertigen Lösungen 0,01 bis 15 % des Wirkstoffs enthalten. Emulsionskonzentrate enthalten 10 bis 50 % und Lösungskonzentrate enthalten in der gebrauchsfertigen Lösung 0,0001 bis 20 % Aktivsubstanz. So kann ein 70 %iges Spritzpulver z.B. aus 70 Teilen des Wirkstoffs, 5 Teilen Natriumdibutylnaphthylsulfonat, dazu 3 Teilen Naphthalinsulfonsäuren - Phenolsulfonsäuren - Formaldehyd-Kondensat (im Mischverhältnis 3:2:1), 10 Teilen Kaolin und 12 Teilen Kreide, z.B. Champagne-Kreide zusammengesetzt sein. Ein 40 %iges Spritzpulver kann z.B.aus folgenden Stoffen bestehen: 40 Teile Wirkstoff, 5 Teile Natrium-Ligninsulfonat, 1 Teil Natrium-Dibutylnaphthylsulfonat und 54 Teile Kieselsäure. Die Herstellung eines 25 %igen Spritzpulvers kann auf unterschiedlicher Art erfolgen. So kann dieses sich z.B. zusammensetzen aus: 25 Teilen der Aktivsubstanz, 4,5 Teilen Calcium-Ligninsulfonat, 1,9 Teilen Kreide, [z.B. Champagne-Kreide]/ Hydroxyethylencellulose-Gemisch (1:1), 1,5 Teilen Natrium-Dibutylnaphthylsulfonat, 19,5 Teilen Kieselsäure, 19,5 Teilen Kreide, [z.B. Champagne-Kreide] und 28,1 Teilen Kaolin. Ein 25 %iges Spritzpulver kann z.B. auch bestehen aus 25 Teilen Wirkstoff, 2,5 Teilen Isooctylphenoxypolyoxyethylen-ethanol, 1,7 Teilen [Champagne]-Kreide/Hydroxyethylcellulosegemisch (1:1), 8,3 Teilen Natriumsilikat, 16,5 Teilen Kieselgur und 46 Teilen Kaolin. Ein 10 %iges Spritzpulver lässt sich z.B. herstellen aus 10 Teilen des Wirkstoffes, 3 Teilen eines Gemisches aus Natriumsalzen von gesättigten Fettalkoholsulfonaten, 5 Teilen Naphthylinsulfonsäure/Formaldehyd-Kondensat und 82 Teilen Kaolin. Andere Spritzpulver können Gemische darstellen aus 5 bis 30 % der Aktivsubstanz zusammen mit 5 Teilen eines aufsaugenden Trägermaterials

wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykolethers. Ein 25 %iges Emulsions-Konzentrat kann z.B. folgende emulgierbare Stoffe enthalten: 25 Teile des Wirkstoffs, 2,5 Teile epoxidiertes Pflanzenöl, 10 Teile eines Alkylarylsulfonat-Fettalkoholpolyglykolether-Gemisches, 5 Teile Dimethylformamid und 57,5 Teile Xylol.

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine Verbindung der Formel I (z.B. 1.1 bis 1.9, 1.13, 1.16, 1.35, 1.38, 1.71 oder 1.78 enthielten, liessen sich mit sehr gutem Erfolg pflanzenwuchsregulatorisch und/oder zur Bekämpfung von phytopathogenen Mikroorganismen einsetzen. Mit gleich gutem oder ähnlichem Erfolg können auch andere Verbindungen aus den Tabellen 1 und 1a eingesetzt werden.

Biologische Beispiele

Die in den nachfolgenden Beispielen verwendeten Spritzbrühen wurden, wie oben beschrieben, formuliert.

Beispiel 4: Wuchshemmung bei Getreide

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge wurden ca. 21 Tage nach der Aussaat mit der wässrigen Spritzbrühe eines Wirkstoffes der Formel I besprüht. Die Wirkstoffmenge betrug umgerechnet 0,5 bzw. 2,5 kg Aktivsubstanz per Hektar. 10 und 21 Tage nach Applikation wurde das Wachstum des Getreides beurteilt. Hierbei konnte festgestellt werden, dass Getreidepflanzen die mit Wirkstoffen der Formel I behandelt worden waren, im Vergleich zu unbehandelten Kontrollpflanzen eine starke Wuchsreduktion aufwiesen. Als besonders wirksam erwiesen sich Verbindungen der Formel I, bei denen der Substituent A für einen durch Halogen substituierten oder unsubstituierten Phenylrest steht, under anderen die Verbindungen Nr. 1.1 bis 1.9, 1.38, 1.71 sowie 2.9.

Beispiel 5: Wuchshemmung bei Gräsern

In Kunststoffschalen mit Erde-Trof-Sand-Gemisch (6:3:1) wurden die Gräser Lolium perenne, Poa pratensis, Festuca orina und Cynodon dactylon im Gewächshaus angesät und nach Bedarf bewässert. Die aufgelaufenen Gräser wurden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge betrug umgerechnet 2,5 kg Aktivsubstanz per Hektar. 10 und 21 Tage nach Applikation wurde das Wachstum der Gräser beurteilt, dabei zeigte es sich, dass die erfindungsgemässen Wirkstoffe aus den Tabellen 1 und 1a eine merkliche Wuchshemmung bewirkten. Besonders deutliche Wuchshemmung wurde unter anderen mit den Verbindungen Nr. 1.1 bis 1.9, 1.38, 1.71 sowie 2.9 erzielt.

Beispiel 6: Ertragssteigerung durch Wachstumsregulierung an Sojabohnen

In Kunststoffbehältern mit einem Erde-Trof-Sandgemisch im Verhältnis 6:3:1 wurden Sojabohnen der Sorte "Hark" angesät und in eine Klimakammer gegeben. Durch optimale Temperaturwahl, Beleuchtung, Düngerzugabe und Bewässerung entwickelten sich die Pflanzen nach ca. 5 Wochen in dem 5-6 Trifola-Blattstadium. Zu diesem Zeitpunkt wurden die Pflanzen mit der wässrigen Brühe eines Wirkstoffes der Formel I bis zur guten Benetzung besprüht. Die Wirkstoffkonzentration betrug 500 ppm Aktivsubstanz. Die Auswertung erfolgte ca. 5 Wochen nach der Applikation des Wirkstoffs. Im Vergleich zu unbehandelten Kontrollpflanzen bewirkten die erfindungsgemässen Wirkstoffe der Formel I eine merkliche Erhöhung der Anzahl und des Gewichts der geernteten Schoten. Als besonders wirksam erwiesen sich die Verbindungen Nr. 1.1 bis 1.9, 1.38, 1.71 sowie 2.9.

Beispiel 7: Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet,

dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Bei der residual-protektiven Behandlung von Gerstenpflanzen gegen Erysiphe-Pilze mit Aktivsubstanzen der Formel I, wie z.B. Verbindungen Nr. 1.1, 1.2, 1.3, 1.4, 1.5, 1.6, 1.7, 1.8, 1.13, 1.16, 1.38 oder 1.71 wurde der Befall der behandelten Pflanzen im Vergleich mit Kontrollpflanzen (100% Befall) auf weniger als 10% zurückgedrängt. Darüberhinaus zeigten die Verbindungen Nr. 1.1, 1.2, 1.3, 1.4, 1.13, 1.38, 1.5, 1.6 und 1.8 eine sehr gute systemische Wirkung. Auch Zwischenprodukte der Formel V und insbesondere der Formel VI (mit G = $OSO_2CH_3$) zeigten in diesen Versuchen fungizide Wirkung.

10-15 cm hohe Erdnusspflanzen wurden mit einer aus Spritzpulver der Wirksubstanz hergestelleten Spritzbrühe (0,02 % Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

Im Vergleich zu unbehandelten Kontrolle (Anzahl und Grösse der Flecken = 100 %) zeigten die Pflanzen, die mit Wirkstoffen der Formel I behandelt waren, einen geringeren oder fast keinen Pilzbefall. So verhinderten unter anderen die Verbindungen Nr. 1.1 bis 1.9, 1.13, sowie 1.38 das Auftreten von Flecken fast vollständig (0 bis 5% Befall).

<u>Beispiel 8</u>: <u>Wirkung gegen Puccinia graminis auf Weizen</u>

a) <u>Residual-protektive Wirkung</u>

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt.

Die Beurteilung der Rostpustelnentwicklung erfolgte 12 Tage nach der Infektion.

Hierbei zeigten unter anderen die Verbindungen Nr. 1.1, 1.2, 1.13, 1.3, 1.5, 1.6 und 1.8 eine gute residual-protektive Wirkung. Sie unterdrückte den Rostpustelnbefall fast vollständig (0 bis 5% Befall).

b) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelentwickung erfolgte 12 Tage nach der Infektion.

Unter anderem bewirkten die Verbindungen Nr. 1.1, 1.2, 1.3, 1.13, 1.38, 1.5 und 1.8 eine fast vollständige Unterdrückung der Rostpustelent - wicklung (0 bis 5% Befall).

Beispiel 9 : Residual protective Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe

(0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen der Formel I zeigten eine gute Wirkung gegen Venturia-Erreger, insbesondere die Verbindungen Nr. 1.1, 1.2, 1.3, 1.13, 1.38, 1.4, 1.5 und 16. hemmten den Schorfbefall im Vergleich zu unbehandelten Kontrollpflanzen auf weniger als 20%. Die Verbindungen Nr. 1.3, 1.4, 1.13, 1.38, und 1.5 erreichten dies sogar noch in einer Dosierung von 0.02%.

Patentansprüche: (für alle benannten Länder ausser Oesterreich)

1. Verbindungen der Formel I

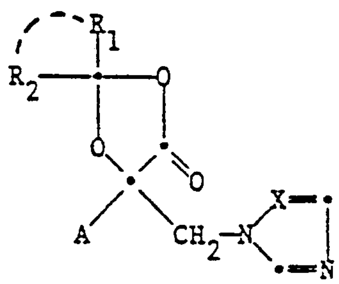

(I)

worin $R_1$ und $R_2$ unabhängig voneinander Wasserstoff, ein gegebenenfalls ein- oder mehrfach durch Halogen, $C_1$-$C_4$-Alkoxy, $C_1$-$C_4$-Alkylthio, Phenyl oder Phenoxy substituiertes $C_1$-$C_8$-Alkyl oder Phenyl bedeuten, wobei die genannten Phenylreste gegebenenfalls ein- oder mehrfach durch Halogen, Nitro, Trifluormethyl, $C_1$-$C_4$-Alkyl oder Methoxy substituiert sind oder $R_1$ und $R_2$ zusammen einen drei- bis siebengliedrigen, carbocyclischen Ring bilden, der gegebenenfalls durch Halogen, $C_1$-$C_4$-Alkyl, $C_1$-$C_4$-Alkoxy oder $C_1$-$C_3$-Alkylthio substituiert ist, A einen gegebenenfalls ein- oder mehrfach durch Halogen, Trifluormethyl, Nitro, $C_1$-$C_4$-Alkyl, $C_1$-$C_4$-Alkoxy oder Phenyl substituierten Phenylrest darstellt, X -CH= oder -N= ist, unter Einschluss ihrer Säureadditionssalze mit organischen und anorganischen Säuren, sowie ihren Metallkomplex-Salzen.

2. Verbindungen der Formel I nach Anspruch 1, dadurch gekennzeichnet, $R_1$ und $R_2$ unabhängig voneinander für Wasserstoff, $C_1$-$C_8$-Alkyl, ein gegebenenfalls durch Halogen, Nitro, Trifluormethyl, Methyl oder Ethyl substituiertes Phenyl oder Benzyl stehen, A ein gegebenenfalls ein- oder mehrfach durch Halogen, Trifluormethyl, Nitro, $C_1$-$C_4$-Alkyl, $C_1$-$C_4$-Alkoxy oder Phenyl substituiertes Phenyl, bedeutet und X für -CH= oder -N= steht.

3. Verbindungen der Formel I nach Anspruch 1, dadurch gekennzeichnet, dass $R_1$ und $R_2$ unabhängig voneinander für Wasserstoff, $C_1$-$C_5$-Alkyl oder Phenyl stehen, A ein gegebenenfalls ein- oder mehrfach durch Halogen, $C_1$-$C_3$-Alkyl, Methoxy oder Phenyl substituiertes Phenyl bedeutet und X für -CH= oder -N= steht.

4. Verbindungen der Formel I nach Anspruch 1, dadurch gekennzeichnet, dass $R_1$ und $R_2$ unabhängig voneinander für $C_1$-$C_5$-Alkyl stehen, A Phenyl, 2,4-Dichlorphenyl, 2,4-Dibromphenyl, 4-Chlorphenyl, 2-Chlor-4-fluorphenyl oder 4-Bromphenyl bedeutet und X für -CH= oder -N= steht.

5. Verbindungen der Formel I nach Anspruch 1, dadurch gekennzeichnet, dass $R_1$ und $R_2$ zusammen einen drei- bis siebengliedrigen, gegebenenfalls durch Methoxy substituierten, carbocyclischen Ring bilden.

6. Die 1,2,4-Triazolyl-derivate der Formel I nach Anspruch 1.

7. Eine Verbindung der Formel I nach Anspruch 1, ausgewählt aus der Gruppe:
4-(1H-1,2,4-Triazolylmethyl)-4-(4-chlorphenyl)-2,2-dimethyl-1,3-dioxolan-5-on,

4-(1H-1,2,4-Triazolylmethyl)-4-(4-bromphenyl)-2,2-dimethyl-1,3-dioxolan-5-on,

4-(1H-1,2,4-Triazolylmethyl)-4-(phenyl)-2,2-dimethyl-1,3-dioxolan-5-on,

4-(1H-1,2,4-Triazolylmethyl)-4-(2,4-dichlorphenyl)-2,2-dimethyl-1,3-dioxolan-5-on,

4-(1H-Imidazolylmethyl)-4-(4-chlorphenyl)-2,2-dimethyl-1,3-dioxolan-5-on,

4-(1H-1,2,4-Triazolylmethyl)-4-(4-chlorphenyl)-2,2-diethyl-1,3-dioxolan-5-on,

4-(1H-1,2,4-Triazolylmethyl)-5-(2-chlor-4-fluorphenyl)-2,2-dimethyl-1,3-dioxolan-5-on.

8. Verfahren zur Herstellung von Verbindungen der Formel I nach Anspruch 1, dadurch gekennzeichnet, dass man ein Dioxolanon der Formel VI

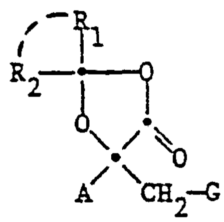

(VI)

mit einer Verbindung der Formel 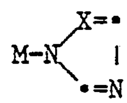

zur Reaktion bringt oder, dass man eine Verbindung der Formel V

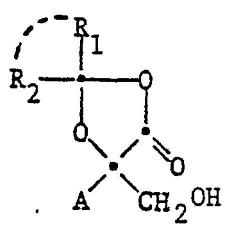

(V)

mit N,N'-Thiocarbonyl-1H-1,2,4-triazol oder N,N'-Thiocarbonyl-1H-imidazol umsetzt, wobei $R_1$, $R_2$, A und X die unter Formel I angegebenen Bedeutungen haben, G für eine der üblichen Abgangsgruppen steht und M Wasserstoff oder ein Metallkation bedeutet.

9. Verfahren nach Anspruch 8, dadurch gekennzeichnet, dass der Substituent G in Formel VI für Acyloxy, Arylsulfonyloxy, Alkylsulfonyloxy oder Halogen steht.

10. Verfahren nach Anspruch 8, dadurch gekennzeichnet, dass der Substituente G in Formel VI für Chlor oder Brom steht.

11. Verfahren nach Anspruch 8, dadurch gekennzeichnet, dass man die Reaktion in Gegenwart einer Base durchführt.

12. Verfahren nach Anspruch 8, dadurch gekennzeichnet, dass man die Reaktion in einem üblichen , inerten organischen Lösungsmittel durchführt.

13. Verfahren nach Anspruch 8, dadurch gekennzeichnet, dass man eine Verbindung der Formel I gemäss einem der Ansprüche 2 bis 8 herstellt.

14. Mittel zur Regulierung des Pflanzenwachstums und/oder zur Bekämpfung und/oder Verhütung eines Befalls von Mikroorganismen, dadurch gekennzeichnet, dass es als mindestens eine aktive Komponente eine Verbindung der Formel I nach Anspruch 1 enthält.

15. Mittel nach Anspruch 14, gekennzeichnet durch den Gehalt an 0,01 bis 90 Gewichtsprozent einer Verbindung der Formel I nach Anspruch 1, 99,99 bis 10% Gewichtsprozent an Zuschlagstoffen und unter diesen 0 bis 30 Gewichtsprozent eines Tensides.

16. Mittel nach einem der Ansprüche 14 oder 15, dadurch gekennzeichnet, dass es als mindestens eine aktive Komponente eine Verbindung der Formel I nach einem der Ansprüche 2 bis 8 enthält.

17. Verfahren zur Herstellung von Mitteln zur Regulierung des Pflanzenwachstums und/oder zur Bekämpfung und/oder Verhütung eines Befalls von Mikroorganismen, dadurch gekennzeichnet, dass man eine Verbindung der Formel I nach einem der Ansprüche 1 bis 8, zusammen mit geeigneten Trägerstoffen und/oder Tensiden innig vermischt.

18. Verwendung von Verbindungen der Formel I nach Anspruch 1 zur Regulierung des vegetativen Wachstums bei Pflanzen und/oder zur Bekämpfung und/oder zur Verhütung eines Befalls von phytopathogenen Pilzen bei Kulturpflanzen.

19. Verwendung nach Anspruch 18 von Verbindungen der Formel I, gemäss einem der Ansprüche 2 bis 8.

20. Verwendung nach Anspruch 18 zur Wuchshemmung von Getreide- oder Grassorten.

21. Verwendung nach Anspruch 18 zur Ertragssteigerung in Getreide-, Tabak-, Soja-, Zuckerrüben-, Mais- oder Reiskulturen.

22. Verwendung nach Anspruch 18 zur Erhöhung der Knickfestigkeit von Weizen, Roggen, Gerste, Hafer oder Reis.

23. Die Verbindungen der Formel V

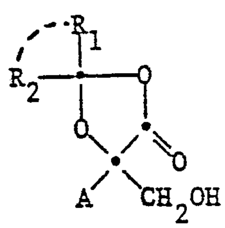

(V)

worin $R_1$ und $R_2$ unabhängig voneinander Wasserstoff, ein gegebenenfalls ein- oder mehrfach durch Halogen, $C_1$-$C_4$-Alkoxy, $C_1$-$C_4$-Alkylthio, Phenyl oder Phenoxy substituiertes $C_1$-$C_8$-Alkyl oder Phenyl bedeuten, wobei die genannten Phenylreste gegebenenfalls ein- oder mehrfach durch Halogen, Nitro, Trifluormethyl, $C_1$-$C_4$-Alkyl oder Methoxy substituiert sind oder $R_1$ und $R_2$ zusammen einen drei- bis siebengliedrigen, carbocyclischen Ring bilden, der gegebenenfalls

durch Halogen, $C_1$-$C_4$-Alkyl, $C_1$-$C_4$-Alkoxy oder $C_1$-$C_3$-Alkylthio substituiert ist und A einen gegebenenfalls ein- oder mehrfach durch Halogen, Trifluormethyl, Nitro, $C_1$-$C_4$-Alkyl, $C_1$-$C_4$-Alkoxy oder Phenyl substituierten Phenylrest darstellt.

24. Die Verbindungen der Formel VI

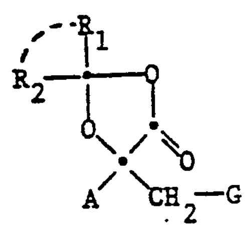

(VI)

worin $R_1$ und $R_2$ unabhängig voneinander Wasserstoff, ein gegebenenfalls ein- oder mehrfach durch Halogen, $C_1$-$C_4$-Alkoxy, $C_1$-$C_4$-Alkylthio, Phenyl oder Phenoxy substituiertes $C_1$-$C_8$-Alkyl oder Phenyl bedeuten, wobei die genannten Phenylreste gegebenenfalls ein- oder mehrfach durch Halogen, Nitro, Trifluormethyl, $C_1$-$C_4$-Alkyl oder Methoxy substituiert sind oder $R_1$ und $R_2$ zusammen einen drei- bis siebengliedrigen, carbocyclischen Ring bilden, der gegebenenfalls durch Halogen, $C_1$-$C_4$-Alkyl, $C_1$-$C_4$-Alkoxy oder $C_1$-$C_3$-Alkylthio substituiert ist, A einen gegebenenfalls ein- oder mehrfach durch Halogen, Trifluormethyl, Nitro, $C_1$-$C_4$-Alkyl, $C_1$-$C_4$-Alkoxy oder Phenyl substituierten Phenylrest darstellt und G für eine übliche Abgangsgruppe steht.

25. Verbindungen der Formel VI nach Anspruch 24, dadurch gekennzeichnet, dass G für $C_1$-$C_6$-Acyloxy, Benzolsulfonyloxy, p-Brombenzolsulfonyloxy, p-Tosyloxy, $C_1$-$C_6$-Alkylsulfonyloxy, Chlor oder Brom steht.

Patentansprüche: (für Oesterreich)

1. Mittel zur Regulierung des Pflanzenwachstums und/oder zur Bekämpfung und/oder Verhütung eines Befalls von Mikroorganismen, dadurch gekennzeichnet, dass es als mindestens eine aktive Komponente eine Verbindung der Formel I

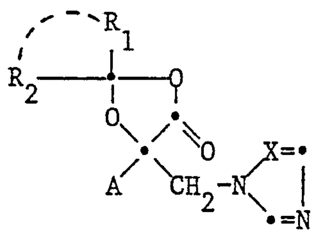

(I)

enthält, worin $R_1$ und $R_2$ unabhängig voneinander Wasserstoff, ein gegebenenfalls ein- oder mehrfach durch Halogen, $C_1$-$C_4$-Alkoxy, $C_1$-$C_4$-Alkylthio, Phenyl oder Phenoxy substituiertes $C_1$-$C_8$-Alkyl oder Phenyl bedeuten, wobei die genannten Phenylreste gegebenenfalls ein- oder mehrfach durch Halogen, Nitro, Trifluormethyl, $C_1$-$C_4$-Alkyl oder Methoxy substituiert sind oder $R_1$ und $R_2$ zusammen einen drei- bis siebengliedrigen, carbocyclischen Ring bilden, der gegebenenfalls durch Halogen, $C_1$-$C_4$-Alkyl, $C_1$-$C_4$-Alkoxy oder $C_1$-$C_3$-Alkylthio substituiert ist, A einen gegebenenfalls ein- oder mehrfach durch Halogen, Trifluormethyl, Nitro, $C_1$-$C_4$-Alkyl, $C_1$-$C_4$-Alkoxy oder Phenyl substituierten Phenylrest darstellt, X -CH= oder -N= ist, unter Einschluss ihrer Säureadditionssalze mit organischen und anorganischen Säuren, sowie ihren Metallkomplex-Salzen.

2. Mittel nach Anspruch 1, gekennzeichnet durch den Gehalt an 0,01 bis 90 Gewichtsprozent einer Verbindung der Formel I nach Anspruch 1, 99,99 bis 10% Gewichtsprozent an Zuschlagstoffen und unter diesen 0 bis 30 Gewichtsprozent eines Tensides.

3. Mittel nach einem der Ansprüche 1 oder 2, dadurch gekennzeichnet, dass es als aktive Komponente eine Verbindung der Formel I enthält, worin $R_1$ und $R_2$ unabhängig voneinander für Wasserstoff, $C_1$-$C_8$-Alkyl, ein gegebenenfalls durch Halogen, Nitro, Trifluormethyl, Methyl oder Ethyl substituiertes Phenyl oder Benzyl stehen, A ein gegebenenfalls ein- oder mehrfach durch Halogen, Trifluormethyl, Nitro, $C_1$-$C_4$-Alkyl, $C_1$-$C_4$-Alkoxy oder Phenyl substituiertes Phenyl, bedeutet und X für -CH= oder -N= steht.

4. Mittel nach einem der Ansprüche 1 oder 2, dadurch gekennzeichnet, dass es als aktive Komponente eine Verbindung der Formel I enthält, worin $R_1$ und $R_2$ unabhängig voneinander für Wasserstoff, $C_1$-$C_5$-Alkyl oder Phenyl stehen, A ein gegebenenfalls ein- oder mehrfach durch Halogen, $C_1$-$C_3$-Alkyl, Methoxy oder Phenyl substituiertes Phenyl bedeutet und X für -CH= oder -N= steht.

5. Mittel nach einem der Ansprüche 1 oder 2, dadurch gekennzeichnet, dass es als aktive Komponente eine Verbindung der Formel I enthält, worin $R_1$ und $R_2$ unabhängig voneinander für $C_1$-$C_5$-Alkyl stehen, A Phenyl, 2,4-Dichlorphenyl, 2,4-Dibromphenyl, 4-Chlorphenyl, 2-Chlor-4-fluorphenyl oder 4-Bromphenyl bedeutet und X für -CH= oder -N= steht.

6. Mittel nach einem der Ansprüche 1 oder 2, dadurch gekennzeichnet, dass es als aktive Komponente eine Verbindung der Formel I enthält, worin $R_1$ und $R_2$ zusammen einen drei- bis siebengliedrigen, gegebenenfalls durch Methoxy substituierten, carbocyclischen Ring bilden.

7. Mittel nach einem der Ansprüche 1 oder 2, dadurch gekennzeichnet, dass es als aktive Komponente ein 1,2,4-Triazolyl-derivat im Umfang der in Anspruch 1 definierten Formel I enthält.

8. Mittel nach einem der Ansprüche 1 oder 2, dadurch gekennzeichnet, dass es als aktive Komponente eine Verbindung der Formel I, ausgewählt aus der Reihe:

4-(1H-1,2,4-Triazolylmethyl)-4-(4-chlorphenyl)-2,2-dimethyl-1,3-dioxolan-5-on,

4-(1H-1,2,4-Triazolylmethyl)-4-(4-bromphenyl)-2,2-dimethyl-1,3-dioxolan-5-on,

4-(1H-1,2,4-Triazolylmethyl)-4-(phenyl)-2,2-dimethyl-1,3-dioxolan-5-on,

4-(1H-1,2,4-Triazolylmethyl)-4-(2,4-dichlorphenyl)-2,2-dimethyl-1,3-dioxolan-5-on,

4-(1H-Imidazolylmethyl)-4-(4-chlorphenyl)-2,2-dimethyl-1,3-dioxolan-5-on,

4-(1H-1,2,4-Triazolylmethyl)-4-(4-chlorphenyl)-2,2-diethyl-1,3-dioxolan-5-on oder

4-(1H-1,2,4-Triazolylmethyl)-5-(2-chlor-4-fluorphenyl)-2,2-dimethyl-1,3-dioxolan-5-on enthält.

9. Verfahren zur Herstellung von Wirkstoffen der Formel I nach Anspruch 1, dadurch gekennzeichnet, dass man ein Dioxolanon der Formel VI

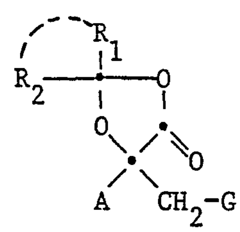

(VI)

mit einer Verbindung der Formel 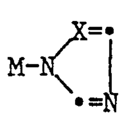

zur Reaktion bringt oder, dass man eine Verbindung der Formel V

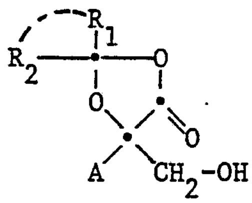

(V)

mit N,N'-Thiocarbonyl-1H-1,2,4-triazol oder N,N'-Thiocarbonyl-1H-imidazol umsetzt, wobei $R_1$, $R_2$, A und X die unter Formel I angegebenen Bedeutungen haben, G für eine der üblichen Abgangsgruppen steht und M Wasserstoff oder ein Metallkation bedeutet.

10. Verfahren nach Anspruch 9, dadurch gekennzeichnet, dass der Substituent G in Formel VI für Acyloxy, Arylsulfonyloxy, Alkylsulfonyloxy oder Halogen steht.

11. Verwendung von Verbindungen der Formel I nach Anspruch 1 zur Regulierung des vegetativen Wachstums bei Pflanzen und/oder zur Bekämpfung und/oder zur Verhütung eines Befalls von phytopathogenen Pilzen bei Kulturpflanzen.

12. Verwendung nach Anspruch 11 von Verbindungen der Formel I, gemäss einem der Ansprüche 2 bis 8.

13. Verwendung nach Anspruch 11 zur Wuchshemmung von Getreide- oder Grassorten.

14. Verwendung nach Anspruch 11 zur Ertragssteigerung in Getreide-, Tabak-, Soja-, Zuckerrüben-, Mais- oder Reiskulturen.

15. Verwendung nach Anspruch 11 zur Erhöhung der Knickfestigkeit von Weizen, Roggen, Gerste, Hafer oder Reis.